# EUROPEAN PATENT APPLICATION

(11) **EP 4 793 813 A1**
(43) Date of publication of application: **19.08.2026**
(21) Application number: 25720619.3
(22) Date of filing: 24.03.2025
(51) Int. Cl.: G06F 30/20, G16C 60/00, G06F 119/14, G06F 119/08, G06F 111/10

(54) **NUMERICAL REACTOR, METHOD FOR VERIFYING REACTOR DESIGN BY USING NUMERICAL REACTOR, AND METHOD FOR PREDICTING REACTOR OPERATION**

(30) Priority: 13.12.2024 CN 202411844651
(71) Applicant: China Institute of Atomic Energy, Beijing 102413 (CN); University Of Science And Technology Beijing, Beijing 100083 (CN); Computer Network Information Center Chinese Academy of Sciences, Beijing 100083 (CN)
(72) Inventor: LIU, Tiancai, Beijing 102413 (CN); YANG, Wen, Beijing 102413 (CN); HU, Changjun, Beijing 102413 (CN); HE, Xinfu, Beijing 102413 (CN); WANG, Jue, Beijing 102413 (CN); HU, Yun, Beijing 102413 (CN); CAO, Jinli, Beijing 102413 (CN); WANG, Xuesong, Beijing 102413 (CN); JIANG, Qiang, Beijing 102413 (CN); CHENG, Daoxi, Beijing 102413 (CN); WU, Mingyu, Beijing 102413 (CN)
(74) Representative: Zimmermann, Tankred Klaus
(86) International application number: PCT/CN2025/084472
(87) International publication number: WO 2026/123498

(57) **Abstract**

Embodiments of the present disclosure relate to a field of reactor numerical simulation technology, and in particular to a numerical reactor, a method of verifying a reactor design using the numerical reactor, and a method of predicting a reactor operation using the numerical reactor. The numerical reactor includes: a physical-thermal structure module, configured to simulate a neutron field, a temperature field and a stress field between cores of a reactor; a fuel-material module, configured to simulate a microstructural change of a fuel in the reactor during a fission process and a microstructural change of a material of the reactor; a coupling module, configured to couple a neutron field, a temperature field and a stress field of the fuel-material module according to the neutron field, the temperature field and the stress field of the physical-thermal structure module, and configured to couple the neutron field, the temperature field and the stress field of the physical-thermal structure module according to the microstructural change of the material and the microstructural change of the fuel in the fuel-material module. The numerical reactor according to embodiments of the present disclosure may ensure accuracy and reliability of the determined microstructural changes of the fuel and the material as well as the neutron field, the temperature field and the stress field between cores.

## Description

### TECHNICAL FIELD

Embodiments of the present disclosure relates to a field of reactor numerical simulation technology, and in particular to a numerical reactor, a method of verifying a reactor design using the numerical reactor, and a method of predicting a reactor operation using the numerical reactor.

### BACKGROUND

The statements here merely provide background information related to the present disclosure and do not necessarily constitute prior art.

In the current field of nuclear energy utilization, nuclear reactor engineering is a complex project involving multiple disciplines such as neutron physics, thermal hydraulics, system analysis, mechanics, and irradiation effects. Research and analysis of the nuclear reactor engineering generally require the use of research tools, and a numerical reactor is an important research tool. The numerical reactor plays a crucial role in deeply understanding physical characteristics of a nuclear reactor, optimizing a design, and ensuring a safe operation of a nuclear reactor.

However, current numerical reactors face many challenges in practical applications. Due to complexity and uncertainty of a reactor system, an established numerical reactor generally has a low accuracy and is difficult to fully and accurately reflect an actual operation of a reactor. For example, in the invention patent with publication number CN117454627A, a numerical reactor nuclear-thermal-material coupling simulation method based on a unified grid is disclosed. In this method, neutron physics, flow heat transfer and material corrosion models are integrated to achieve unified solution of the three physical processes under the same solver, so as to perform a high-fidelity simulation of multi-physical processes in the nuclear reactor core. This method comprehensively considers an impact of oxidation and corrosion of a cladding material on reactor safety. However, it does not involve a mutual influence between a change in properties of the cladding material before and after irradiation and a change in service properties of the fuel before and after service, and it is difficult to ensure an accuracy of the determined corrosion status of the cladding material.

### SUMMARY

A brief overview of the present disclosure is given below in order to provide a basic understanding of some aspects of the present disclosure. It should be understood that the overview is not an exhaustive overview of the present disclosure. It is not intended to determine a key or important part of the present disclosure, nor is it intended to limit the scope of the present disclosure. Its purpose is merely to present some concepts in a simplified form as a prelude to a more detailed description to be described later.

In a first aspect, embodiments of the present disclosure provide a numerical reactor, including: a physical-thermal structure module, configured to simulate a neutron field, a temperature field and a stress field between cores of a reactor; a fuel-material module, configured to simulate a microstructural change of a fuel in the reactor during a fission process and a microstructural change of a material of the reactor; a coupling module, configured to couple a neutron field, a temperature field and a stress field of the fuel-material module according to the neutron field, the temperature field and the stress field of the physical-thermal structure module, and configured to couple the neutron field, the temperature field and the stress field of the physical-thermal structure module according to the microstructural change of the material and the microstructural change of the fuel in the fuel-material module.

According to embodiments of the present disclosure, a multi-scale simulation is performed by the physical-thermal structure module and the fuel-material module, and the simulation results are coupled by the coupling module. The neutron field, the temperature field and the stress field of the fuel-material module may be determined based on determined neutron field, temperature field and stress field. Further, the neutron field, the temperature field and the stress field of the physical-thermal structure module may be determined based on the neutron field, the temperature field and the stress field of the fuel-material module, thereby ensuring the accuracy and reliability of the ultimately determined microstructural change of the fuel during the fission process, the microstructural change of the material, and the neutron field, the temperature field and the stress field between cores of the reactor.

On a second aspect, embodiments of the present disclosure further provide a method of verifying a reactor design, including: inputting obtained design parameters of a reactor into the numerical reactor provided in embodiments of the present disclosure; allowing the numerical reactor to operate according to the design parameters to obtain an operation result; and comparing the operation result with an expected result to determine whether the design parameters meet an expected design objective.

On a third aspect, embodiments of the present disclosure further provide a method of predicting a reactor operation, including: obtaining current actual operation data of the reactor; inputting the current actual operation data into the numerical reactor provided in embodiments of the present disclosure; allowing the numerical reactor to operate according to the current actual operation data to obtain an operation result for a predetermined future time; and predicting a future operation status of the reactor according to the obtained operation result.

The above and other advantages of the present disclosure will become more apparent through the following detailed description of preferred embodiments of the present disclosure with reference to the accompanying drawings.

### BRIEF DESCRIPTION OF THE DRAWINGS

In order to further illustrate the above and other advantages and features of the present disclosure, specific embodiments of the present disclosure will be further described in detail below with reference to the accompanying drawings. The accompanying drawings, together with the following detailed description, are incorporated into and form a part of this specification. Elements having the same function and structure are denoted by the same reference numeral. It should be understood that the accompanying drawings only depict typical examples of the present disclosure and should not be considered as limiting the scope of the present disclosure.
FIG. 1 shows a schematic structural diagram of a numerical reactor according to an embodiment of the present disclosure;
FIG. 2 shows a schematic diagram of a specific structure of the numerical reactor shown in FIG. 1;
FIG. 3 shows a schematic structural diagram of a numerical reactor according to another embodiment of the present disclosure;
FIG. 4 shows a schematic diagram of correcting parameters of a material irradiation sub-module and a fuel service property sub-module according to an embodiment of the present disclosure;
FIG. 5 shows a schematic diagram of a verification process of a verification module according to an embodiment of the present disclosure;
FIG. 6 shows a schematic diagram of multi-scale physical coupling according to an embodiment of the present disclosure;
FIG. 7 shows a schematic flowchart of a method of verifying a reactor design according to an embodiment of the present disclosure; and
FIG. 8 shows a schematic flowchart of a method of predicting a reactor operation according to an embodiment of the present disclosure.

It should be noted that the accompanying drawings are not necessarily drawn to scale, but are merely shown in a schematic manner that does not affect the reader's understanding.

### Description of reference numerals:

10. Physical-thermal structure module;
20. Fuel-material module; 21. Material irradiation sub-module; 211. Material neutron field; 212. Material stress field; 213. Material temperature field; 22. Fuel service property sub-module;
30. Coupling module;
40. Neutron physics module;
50. Data-driven module; 51. Mapping relationship;
60. Verification module;
71. Measured data; 72. Prior physical knowledge.

### DETAILED DESCRIPTION OF EMBODIMENTS

Exemplary embodiments of the present disclosure will be described below in conjunction with the accompanying drawings. For the sake of clarity and conciseness, not all features of the actual implementations are described in the specification. However, it should be understood that it is necessary to make many implementation-specific determinations, for example, to comply with system and business-related restrictions, during a development of any such actual implementation in order to achieve developer's specific objectives. These restrictions may vary with different implementations. In addition, it should be understood that although the development work may be very complicated and time-consuming, such development work is only a routine task for those skilled in the art who benefit from the content of the present disclosure.

Here, it should be further noted that, in order to avoid obscuring the present disclosure due to unnecessary details, only the device structure and/or processing steps closely related to the solution according to the present disclosure are shown in the accompanying drawings, and other details of little relevance to the present disclosure are omitted.

It should be noted that, unless otherwise defined, technical terms or scientific terms used in the present disclosure should have the common meanings understood by persons with ordinary skills in the field to which the present disclosure belongs.

In the description of the embodiments of the present disclosure, "plurality" means at least two, such as two, three, etc., unless otherwise clearly and specifically defined.

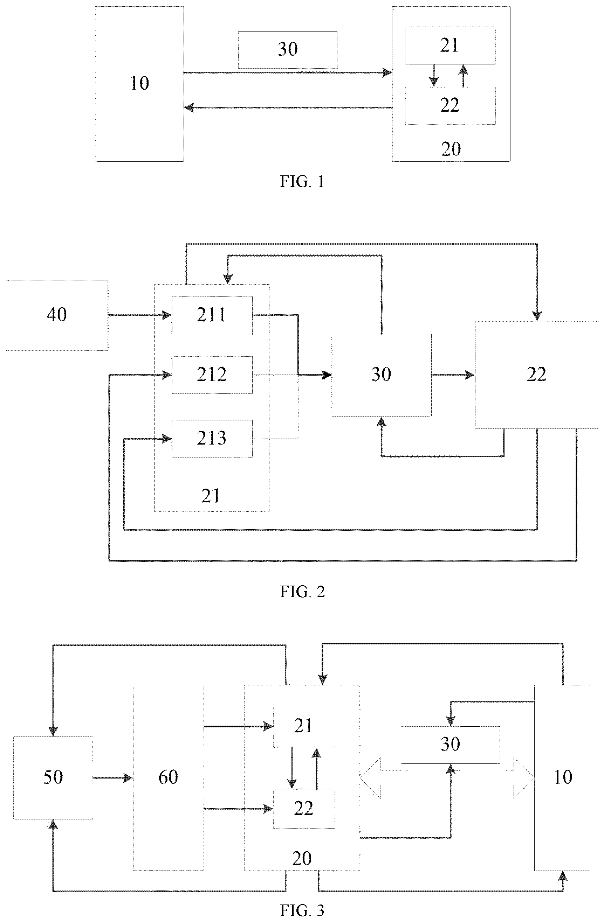

Embodiments of the present disclosure provide a numerical reactor. FIG. 1 shows a schematic structural diagram of a numerical reactor according to an embodiment of the present disclosure. As shown in FIG. 1, the numerical reactor may include at least a physical-thermal structure module 10, a fuel-material module 20 and a coupling module 30.

The physical-thermal structure module 10 is configured to simulate a neutron field, a temperature field and a stress field between cores of a reactor.

The fuel-material module 20 is configured to simulate a microstructural change of a fuel in the reactor during a fission process and a microstructural change of a material in the reactor.

The coupling module 30 is configured to couple a neutron field, a temperature field and a stress field of the fuel-material module 20 according to the neutron field, the temperature field and the stress field of the physical-thermal structure module 10, and configured to couple the neutron field, the temperature field and the stress field of the physical-thermal structure module 10 according to the microstructural change of the material and the microstructural change of the fuel in the fuel-material module 20.

According to embodiments of the present disclosure, a multi-scale simulation is performed by the physical-thermal structure module 10 and the fuel-material module 20, and the simulation results are coupled by the coupling module 30. The neutron field, the temperature field and the stress field of the fuel-material module 20 may be determined based on determined neutron field, temperature field and stress field. Further, the neutron field, the temperature field and the stress field of the physical-thermal structure module 10 may be determined based on the neutron field, the temperature field and the stress field of the fuel-material module 20, thereby ensuring the accuracy and reliability of the ultimately determined microstructural change of the fuel during the fission process, the microstructural change of the material, and the neutron field, the temperature field and the stress field between cores of the reactor.

In some embodiments, the neutron field, the temperature field and the stress field of the fuel-material module 20 are coupled according to the neutron field, the temperature field and the stress field of the physical-thermal structure module 10 by using: ${Φ}_{f} \left(r,t+Δt\right) = {f}_{ϕ} \left({T}_{f}\left(r, t\right),{σ}_{f}\left(r, t\right),{Φ}_{c}\left(r, t\right)\right) ;$ ${T}_{f} \left(r,t+Δt\right) = {f}_{T} \left({T}_{f}\left(r, t\right),{σ}_{f}\left(r, t\right),{Φ}_{f}\left(r, t\right),{T}_{c}\left(r, t\right)\right) ;$ ${σ}_{f} \left(r,t+Δt\right) = {f}_{σ} \left({T}_{f}\left(r, t\right),{σ}_{f}\left(r, t\right),{Φ}_{f}\left(r, t\right),{σ}_{c}\left(r, t\right)\right) ;$ where Φ*_{c}*(*r,t*) represents the neutron field between cores of the physical-thermal structure module 10, *T_{c}*(*r,t*) represents the temperature field between cores of the physical-thermal structure module 10, *σ_{c}*(*r,t*) represents the stress field between cores of the physical-thermal structure module 10, Φ*_{f}*(*r,t*) represents the neutron field of the fuel-material module 20, *T_{f}*(*r,t*) represents the temperature field of the fuel-material module 20, *σ_{f}*(*r,t*) represents the stress field of the fuel-material module 20, Φ*_{f}*(*r,t+*Δ*t*) represents a coupled neutron field of the fuel-material module 20, *T_{f}*(*r,t*+Δ*t*) represents a coupled temperature field of the fuel-material module 20, *σ_{f}*(*rₜ+*Δ*ₜ*) represents a coupled stress field of the fuel-material module 20, *r* represents spatial coordinates, *r* ≤ *r_{c} , r_{c}* represents coordinates of an outer boundary of a cladding of the reactor, *t* represents time, Δ*t* represents a time increment in an iterative calculation, *f_{ϕ}* represents a function for determining the neutron field of the fuel-material module 20 by a numerical method, *f_{T}* represents a function for determining the temperature field of the fuel-material module 20 by a numerical method, and *f_{σ}* represents a function for determining the stress field of the fuel-material module 20 by a numerical method.

According to embodiments of the present disclosure, the neutron field, the temperature field and the stress field of the fuel-material module 20 are coupled by using the neutron field, the temperature field and the stress field of the physical-thermal structure module 10 in the above-mentioned manner, which has a good coupling effect and may ensure the accuracy of the determined neutron field, temperature field and stress field of the fuel-material module 20.

In some embodiments, FIG. 2 shows a schematic diagram of a specific structure of the numerical reactor shown in FIG. 1. As shown in FIG. 1, the fuel-material module 20 includes a material irradiation sub-module 21 and a fuel service property sub-module 22. The material irradiation sub-module 21 is configured to simulate a change in properties of the material caused by the microstructural change of the material of the reactor generated after irradiation. The fuel service property sub-module 22 is configured to simulate the microstructural change of the fuel in the reactor during the fission process.

According to embodiments of the present disclosure, a multi-scale simulation is performed by the material irradiation sub-module 21 and the fuel service property sub-module 22, which may improve the accuracy and reliability of the ultimately determined changes in properties of the material and in service properties of the fuel.

In some embodiments, the coupling module 30 is further configured to couple the service properties of the fuel according to the microstructural change of the material, and couple the properties of the material according to the microstructural change of the fuel. The service properties of the fuel may be determined based on the microstructural change of the material, and the properties of the material may be determined based on the microstructural change of the fuel, so as to improve the accuracy and reliability of the ultimately determined service properties of the fuel and the properties of the material.

**In** some embodiments, the neutron field, the temperature field and the stress field of the fuel-material module 20 and a microstructure of the fuel meet: ${G}_{f} \left(r,t+Δt\right) = {f}_{Gf} \left({Φ}_{f}\left(r, t\right),{T}_{f}\left(r, t\right),{σ}_{f}\left(r, t\right),{G}_{f}\left(r, t\right),{G}_{m}\left(r, t\right)\right) ;$ where *G_{f}*(*r,t*+Δ*t*) represents a coupled microstructure of the fuel, *f_{Gf}* represents a function for determining the microstructure of the fuel by a numerical method, Φ*_{f}*(*r,t*) represents the neutron field of the fuel-material module 20, *T_{f}*(*r,t*) represents the temperature field of the fuel-material module 20, *σ_{f}*(*r,t*) represents the stress field of the fuel-material module 20, *G_{f}*(*r,t*) represents the microstructure of the fuel at time *t* , *Gₘ*(*r,t*) represents the microstructure of the material at time *t* , and Δ*t* represents a time increment in an iterative calculation.

In some embodiments, the neutron field, the temperature field and the stress field of the fuel-material module 20 and a microstructure of the material meet: ${G}_{m} \left(r,t+Δt\right) = {f}_{Gm} \left({Φ}_{f}\left(r, t\right),{T}_{f}\left(r, t\right),{σ}_{f}\left(r, t\right),{G}_{f}\left(r, t\right),{G}_{m}\left(r, t\right)\right) ;$ where *Gₘ*(*r,t*+Δ*t*) represents a coupled microstructure of the material, *f_{Gm}* represents a function for determining the microstructure of the material by a numerical method, Φ*_{f}*(*r,t*) represents the neutron field of the fuel-material module 20, *T_{f}*(*r,t*) represents the temperature field of the fuel-material module 20, *σ_{f}*(*r,t*) represents the stress field of the fuel-material module 20, *G_{f}*(*r,t*) represents the microstructure of the fuel at time *t , Gₘ*(*r,t*) represents the microstructure of the material at time *t,* and Δ*t* represents a time increment in an iterative calculation.

In some embodiments, the change in properties of the material includes a change in macroscopic mechanical properties or in geometric integrity properties of the material. As shown in FIG. 2, the material irradiation sub-module 21 is further configured to transmit the change in the macroscopic mechanical properties or in the geometric integrity properties of the material determined by simulation to the fuel service property sub-module 22, and the fuel service property sub-module 22 is further configured to correct a change in service properties of the fuel according to the change in the macroscopic mechanical properties or in the geometric integrity properties of the material.

According to embodiments of the present disclosure, the material irradiation sub-module 21 may determine the change in the macroscopic mechanical properties or in the geometric integrity properties of the material and transmit the change to the fuel service property sub-module 22, and the fuel service property sub-module 22 may correct the change in the service properties of the fuel according to the change in the macroscopic mechanical properties or in the geometric integrity properties of the material, so that the service properties of the fuel during the service process may be determined accurately.

In some embodiments, when the material irradiation sub-module 21 performs the function of transmitting the change in the macroscopic mechanical properties or in the geometric integrity properties of the material determined by simulation to the fuel service property sub-module 22 and the fuel service property sub-module 22 performs the function of correcting the change in the service properties of the fuel according to the change in the macroscopic mechanical properties or in the geometric integrity properties of the material, the functions may be performed cyclically until a first predetermined duration is reached.

In some embodiments, the service properties of the fuel include fission properties and a fission product behavior, the fission properties include a temperature and stress distribution, and the fission product behavior includes a gaseous fission product behavior and a solid fission product behavior. As shown in FIG. 2, the fuel service property sub-module 22 is further configured to transmit the temperature and stress distribution, the gaseous fission product and the solid fission product of the fuel determined by simulation to the material irradiation sub-module 21, and the material irradiation sub-module 21 is configured to determine the change in the macroscopic mechanical properties or in the geometric integrity properties of the material according to the temperature and stress distribution, the gaseous fission product and the solid fission product of the fuel.

According to embodiments of the present disclosure, the fuel service property sub-module 22 may determine the temperature and stress distribution, the gaseous fission product and the solid fission product of the fuel and transmit them to the material irradiation sub-module 21, and the material irradiation sub-module 21 may determine the change in the macroscopic mechanical properties or in the geometric integrity properties of the material according to the temperature and stress distribution, the gaseous fission product and the solid fission product of the fuel, so that the properties of the material after irradiation may be determined accurately.

In some embodiments, when the fuel service property sub-module 22 performs the function of transmitting the temperature and stress distribution, the gaseous fission product and the solid fission product of the fuel determined by simulation to the material irradiation sub-module 21 and the material irradiation sub-module 21 performs the function of determining the change in the macroscopic mechanical properties or in the geometric integrity properties of the material according to the temperature and stress distribution, the gaseous fission product and the solid fission product of the fuel, the functions may be performed cyclically until a second predetermined duration is reached.

In such embodiments, the first predetermined duration and the second predetermined duration may be the same.

In some embodiments, the coupling module 30 is specifically configured to couple the service properties of the fuel according to the change in the macroscopic mechanical properties or in the geometric integrity properties of the material, so as to ensure the reliability of the obtained service properties of the fuel.

In some embodiments, the coupling module 30 is specifically configured to couple the macroscopic mechanical properties or the geometric integrity properties of the material according to the temperature and stress distribution, the gaseous fission product and the solid fission product of the fuel, so as to ensure the reliability of the obtained macroscopic mechanical properties or geometric integrity properties of the material.

In some embodiments, as shown in FIG. 2, the material irradiation sub-module 21 may include a material neutron field 211, a material stress field 212, and a material temperature field 213. The material neutron field 211 and the material temperature field 213 are configured to simulate a change in the material generated under an irradiation of the material neutron field 211 and a temperature of the material temperature field 213. The material stress field 212 and the material temperature field 213 are configured to simulate a change in the material generated under a stress of the material stress field 212 and a temperature of the material temperature field 213. The coupling module 30 is further configured to couple changes in properties of the material according to simulation results of the material neutron field 211, the material stress field 212 and the material temperature field 213.

According to embodiments of the present disclosure, the changes in the material under the material neutron field 211, the material stress field 212 and the material temperature field 213 are simulated, and the simulation results are coupled using the coupling module 30, which may help further improve the accuracy of determining the changes in the material.

In some embodiments, as shown in FIG. 2, the numerical reactor may further include a neutron physics module 40 used to calculate a power density, a nuclide distribution, etc. inside a fuel pellet.

In some embodiments, an input of the material neutron field 211 is derived from an output of the neutron physics module 40.

In some embodiments, an input of the material stress field 212 is derived from an output of the fuel service property sub-module 22.

In some embodiments, an input of the material temperature field 213 is derived from the output of the fuel service property sub-module 22.

In some embodiments, the coupling module 30 may couple the material neutron field 211, the material stress field 212 and the material temperature field 213 of the material irradiation sub-module 21 by changing a generation, diffusion and aggregation process of point defects of the material in the material during a service process. The coupling module 30 is specifically used to: obtain a first concentration of point defects under the material temperature field 213; obtain a second concentration of point defects under the material neutron field 211 and the material temperature field 213; and obtain a third concentration of point defects under the material stress field 212 and the material temperature field 213.

According to embodiments of the present disclosure, the material neutron field 211, the material stress field 212 and the material temperature field 213 of the material irradiation sub-module 21 are coupled by changing the generation, diffusion and aggregation process of point defects of the material during service, so that a good coupling effect is achieved.

In some embodiments, a total concentration of point defects under a coupling of the material neutron field 211, the material stress field 212 and the material temperature field 213, the third concentration of point defects under the material stress field 212 and the material temperature field 213, the first concentration of point defects under the material temperature field 213, and the second concentration of point defects under the material neutron field 211 and the material temperature field 213 may meet: $C = {C}_{1} + {C}_{2} - {C}_{0} ;$ where *C* represents the total concentration of the point defects under the coupling of the material neutron field 211, the material stress field 212 and the material temperature field 213, *C*₀ represents the first concentration of the point defects under the material temperature field 213, *C*₁ represents the second concentration of the point defects under the material neutron field 211 and the material temperature field 213, and *C*₂ represents the third concentration of the point defects under the material stress field 212 and the material temperature field 213.

In some embodiments, the coupling module 30 is further specifically used to: obtain a first diffusion coefficient of the point defects under the material temperature field 213; obtain a second diffusion coefficient of the point defects under the material neutron field 211 and the material temperature field 213; and obtain a third diffusion coefficient of the point defects under the material stress field 212 and the material temperature field 213.

In some embodiments, the second diffusion coefficient of the point defects under the material neutron field 211 and the material temperature field 213, the first diffusion coefficient of the point defects under the material temperature field 213, the second concentration of the point defects under the material neutron field 211 and the material temperature field 213, and the first concentration of the point defects under the material temperature field 213 meet: ${D}_{1} = {D}_{0} \times \frac{{C}_{1}}{{C}_{0}} ;$ where Di represents the second diffusion coefficient of the point defects under the material neutron field 211 and the material temperature field 213, *D*₀ represents the first diffusion coefficient of the point defects under the material temperature field 213, *C*₁ represents the second concentration of the point defects under the material neutron field 211 and the material temperature field 213, and *C*₀ represents the first concentration of the point defects under the material temperature field 213.

In some embodiments, a defect diffusion coefficient under the coupling of the material neutron field 211, the material stress field 212 and the material temperature field 213, the third diffusion coefficient of the point defects under the material stress field 212 and the material temperature field 213, the total concentration of the point defects, and the third concentration of the point defects under the material stress field 212 and the material temperature field 213 meet: $D = {D}_{2} \times \frac{C}{{C}_{2}} ;$ where D represents the defect diffusion coefficient under the coupling of the material neutron field 211, the material stress field 212 and the material temperature field 213, *D*₂ represents the third diffusion coefficient of the point defects under the material stress field 212 and the material temperature field 213, C represents the total concentration of the point defects under the coupling of the material neutron field 211, the material stress field 212 and the material temperature field 213, and *C*₂ represents the third concentration of the point defects under the material stress field 212 and the material temperature field 213.

In some embodiments, the coupling module 30 is further used to: control an evolution of defects by using a governing equation for defect reaction, under the material neutron field 211, the material stress field 212 and the material temperature field 213, where the governing equation is expressed as: $\begin{matrix}\frac{{dC}^{i}}{dt} = {G}^{i} + {\sum }_{j} {J}^{j \to i} - {\sum }_{i} {J}^{i \to k} \\ = {G}^{i} + {\sum }_{j} K \times {C}^{j} \times {C}^{i - j} - {\sum }_{i} K \times {C}^{i} \times {C}^{k - i} \\ = {G}^{i} + {\sum }_{j} n \times \left({D}^{j}+{D}^{i - j}\right) \times {C}^{j} \times {C}^{i - j} - {\sum }_{i} n \times \left({D}^{i}+{D}^{k - i}\right) \times {C}^{i} \times {C}^{k - i} ;\end{matrix}$ where *i , j* and *k* represent defects of different sizes respectively, *Gⁱ* represents a generation term of the point defect *i,* which is input by the material neutron field 211, *K* and *n* represent a defect reaction rate constant and a reaction radius respectively, a second term on a right side of the governing equation represents an increase term resulting from an evolution from defect *j* to defect *i* , a third term on the right side of the governing equation represents a decomposition, absorption or disappearance term of defect *i* , *Cⁱ* represents a concentration of defect *i* in the material, *C^{j}* represents a concentration of defect *j* in the material, *C*^{*i*-*j*} represents a concentration of defects *i - j* in the material, *C^{k-i}* represents a concentration of defects *k-i* in the material, *Dⁱ* represents a diffusion coefficient of defect *i* in the material, *D^{j}* represents a diffusion coefficient of defect *j* in the material, *D^{i-j}* represents a diffusion coefficient of defects *i - j* in the material, *D*^{*k*-*i*} represents a diffusion coefficient of defects *k-i in* the material, the concentrations *Cⁱ, C^{i-j}, C^{k-i}* and the diffusion coefficients *Dⁱ, D^{i-j} , D^{k-i}* of the defects are obtained under the coupling of the material neutron field 211, the material stress field 212 and the material temperature field 213. A defect distribution function for the material under the coupling of the material neutron field 211, the material stress field 212 and the material temperature field 213 is determined based on an evolution of the point defects, and the change in the macroscopic mechanical properties or in the geometric integrity properties of the material is determined based on the defect distribution function.

In some embodiments, the fuel service property sub-module 22 includes a temperature distribution unit, a stress distribution unit, a microstructure evolution unit, a fission gas behavior unit and a solid fission product behavior unit. The temperature distribution unit is used to simulate a change in a fuel temperature distribution of the fuel during a fission reaction. The stress distribution unit is used to simulate a change in a fuel stress distribution of the fuel during the fission reaction. The microstructure evolution unit is used to simulate a change in the microstructure of the fuel during the fission reaction. The fission gas behavior unit is used to simulate a fuel swelling induced by fission gas generated from the fuel during the fission reaction and a change in an amount of gas released into a gas cavity. The solid fission product behavior unit is used to simulate a change in a distribution of solid fission products generated from the fuel during the fission reaction in the fuel.

According to embodiments of the present disclosure, the fuel service property sub-module 22 includes the temperature distribution unit, the stress distribution unit, the microstructure evolution unit, the fission gas behavior unit and the solid fission product behavior unit, and these different units may be used to respectively simulate a change in the fuel temperature field, a change in the fuel stress field, a microstructure evolution, a fission gas behavior and a solid fission product behavior of the fuel, so that a high reliability may be achieved.

In some embodiments, the numerical reactor may further include a thermal hydraulic module used to simulate thermal hydraulics of the reactor, so as to determine a coolant pressure of the reactor and a temperature distribution on an outer surface of a fuel rod cladding.

In some embodiments, an input of the temperature distribution unit is derived from an output of the neutron physics module and the thermal hydraulic module.

In some embodiments, an input of the stress distribution unit is derived from the output of the neutron physics module and the thermal hydraulic module.

In some embodiments, an input of the microstructure evolution unit is derived from the output of the neutron physics module.

In some embodiments, an input of the fission gas behavior unit is derived from the output of the neutron physics module.

In some embodiments, an input of the solid fission product unit is derived from the output of the neutron physics module.

In some embodiments, the coupling module 30 is further used to couple the fission gas behavior, the solid fission product behavior, the microstructure evolution, the change in the fuel stress field and the change in the fuel temperature field, so as to obtain an internal composition, a structure, a thermal conductivity and a change in a geometric integrity of the fuel.

In some embodiments, the fission gas behavior, the solid fission product, the microstructure evolution, the fuel stress field and the fuel temperature field in the fuel service property sub-module 22 may be determined by coupling the temperature distribution unit, the stress distribution unit, the microstructure evolution unit, the fission gas behavior unit and the solid fission product behavior unit.

In some embodiments, the coupling module 30 is specifically used to: update the temperature and stress distribution as well as changes in the microstructure, the fission gas and the solid fission product behavior of the fuel in service at predetermined time intervals during a coupling process, where the temperature distribution unit, the stress distribution unit, the microstructure evolution unit, the fission gas behavior unit and the solid fission product behavior unit may simulate in sequence during the coupling process.

In some embodiments, the temperature distribution unit is specifically used to characterize a temperature distribution of the fuel in a predetermined space by using a three-dimensional heat conduction equation, which is expressed as: $\frac{\partial }{\partial t} \left[{c}_{ν}\left(T, \overline{r}\right)T\left(\overline{r}, t\right)\right] = ∇ \left[k\left(g, T, \overline{r}\right)⋅∇T\left(\overline{r}, t\right)\right] + q \left(\overline{r}, t\right) ;$ where *T* represents a temperature of the fuel; *t* represents time; *r̅* represents coordinates of the fuel rod in the predetermined space; *cᵥ* represents a heat capacity per unit volume; *q* represents a volumetric heat release rate; and *k*(*g*,*T*,*r*) represents a thermal conductivity of the fuel, which is related to an internal chemical composition and microstructure morphology of the fuel.

In some embodiments, the microstructure evolution unit is specifically used to characterize a change in the microstructure of the fuel in the fission reaction, so as to obtain a structural information of the microstructure. The microstructure includes a chemical composition, a phase structure, a grain size, a morphology, and a porosity. An evolution of non-conserved order parameters and conserved order parameters for the phase structure, the grain size, the morphology and the porosity is controlled by a predetermined evolution equation, which is expressed as: $\frac{\partial {φ}_{i}}{\partial t} = - {L}_{i} \left(g, T, σ\right) \frac{δF}{{δφ}_{i}} ;$ $\begin{matrix}\frac{\partial {c}_{i}}{\partial t} = - ∇ ⋅ \left({J}_{T}+{J}_{c}\right) \\ = ∇ ⋅ \left({M}_{i}\left(g, T, σ\right)∇\frac{δF}{{δc}_{i}}-{M}_{T}\left(g, T, σ\right)\frac{δT}{T}\right) .\end{matrix}$ where *φᵢ* represents the non-conserved order parameters for the phase structure, the grain size, the morphology and the porosity, *Lᵢ*(*g,T,σ*) represents a diffusion mobility of the non-conserved order parameters for the fuel, *F* represents a free energy equation for the fuel, *cᵢ* represents the conserved order parameters for the phase structure, the grain size, the morphology and the porosity, *J_{T}* represents a temperature-driven diffusion flux, *J_{c}* represents a free energy-driven diffusion flux, *Mᵢ*(*g,T,σ*) represents a free energy-driven chemical mobility, *MT*(*g,T,σ*) represents a temperature-driven thermodynamic mobility, *t* represents a service time of the fuel, and *T* represents a service temperature of the fuel.

In some embodiments, the predetermined evolution equation may be a reaction-diffusion (Allen-Cahn) equation and a Cahn-Hilliard equation.

In some embodiments, through the calculation of the microstructure evolution unit, it is possible to obtain a fuel microstructure information, denoted as *g*, which includes information on a fuel phase structure *str,* an elemental composition *c* in the fuel, a fuel grain size *r_{g}*, a fuel grain-boundary morphology *geo_{g}* and a porosity *p .* The fuel microstructure information *g* meets: $g = {f}_{g} \left(str, c, {r}_{g}, {geo}_{g}, p\right) + {g}_{fis} ;$ where *g_{fis}* represents a microstructural change caused by the solid fission product, which may be used as an input parameter for the gaseous fission product behavior and solid fission product behavior in the fission reaction.

In some embodiments, the fission gas behavior unit is specifically used to characterize a transmission of fission gas, an induced swelling and a release amount of the fission gas in the fuel, so as to obtain a distribution of fission gas within grains, on grain boundaries and at grain edges, the release amount of fission gas, the induced swelling, and the porosity distribution. The fission gas includes xenon and krypton.

In some embodiments, the distribution of the fission gas within grains, on grain boundaries and at grain edges and the release amount of the fission gas meet: $\frac{{dw}_{rea}}{dt} = q \left(g, T, σ\right) {C}_{f} P + {C}_{e} \frac{dP}{dt} + n \left(g, T, σ\right) {C}_{f} ;$ where *wᵣₑₐ* represents the release amount of the fission gas, *t* represents a service time of the fuel, *q*(*g,T,σ*) represents a migration coefficient of grain-face bubbles to grain edges, *C_{f}* represents a concentration of grain-boundary bubbles, *P* represents a release coefficient of grain-edge bubbles, *Cₑ* represents a concentration of grain-edge bubbles, and *n*(*g,T,σ*) represents a direct release coefficient of grain-face bubbles.

In some embodiments, the distribution of fission gas within grains, on grain boundaries and at grain edges and the swelling induced by the fission gas meet: $Δ {V}_{α} = \frac{4}{3} π {\sum }_{i} {r}_{i}^{3} ;$ where Δ*V_{α}* represents the swelling induced by the fission gas, and *rᵢ* represents a bubble radius with size *i.*

In some embodiments, based on the distribution of fission gas within grains, on grain boundaries and at grain edges, the porosity distribution of the fuel meets: $p \left(\overline{r}\right) = {p}_{0} \left(\overline{r}\right) + p ′ \left({r}_{i}\right) ;$ where *p*(*r̅*) represents the porosity distribution of the fuel, *P*₀(*r̅*) represents an initial porosity distribution of the fuel, and *P'*(*rᵢ*) represents a porosity distribution caused by the fission gas.

In some embodiments, the fission gas may be xenon (Xe) and krypton (Kr), etc.

In some embodiments, the fission gas behavior unit mainly takes into account a generation of fission gas during the fission process. The gas diffuses through fuel grains and forms bubbles, and bubbles coalesce and grow up, which diffuse to grain boundaries and stay in the fuel, causing fuel swelling. Then, as a burnup increases, grain-boundary bubbles may connect to form a release channel for fission gas, and the fission gas may be released into a gap between the fuel and the cladding. A fission gas atomic concentration *C_{g},* an intragranular bubble concentration *C_{b},* a grain-boundary bubble concentration *C_{f}*, and a grain-edge bubble concentration *Cₑ* in the fuel are mainly governed by a bubble evolution governing equation as follows: $\begin{matrix}\frac{{dC}_{g}}{dt} = s - a \left(g, T, σ\right) {C}_{g}^{2} - b \left(g, T, σ\right) {C}_{g} {C}_{b} - \frac{6 {D}_{g}}{{d}_{g}} {\left.\frac{\partial {C}_{g}}{\partial r}\right|}_{r = {d}_{g} / 2} + e \left(g, T, σ\right) {N}_{b} {C}_{b} \\ + h \left(g, T, σ\right) \left[{N}_{f}{C}_{f}+{N}_{e}{C}_{e}\right] ;\end{matrix}$ $\begin{matrix}\frac{{dC}_{b}}{dt} = a \left(g, T, σ\right) {C}_{g}^{2} / {N}_{b} + b \left(g, T, σ\right) {C}_{g} {C}_{b} / {N}_{b} - l \left(g, T, σ\right) {C}_{b} - e \left(g, T, σ\right) {C}_{b} \\ + m \left(g, T, σ\right) {N}_{b} \left({C}_{f}/{N}_{f}+{C}_{e}/{N}_{e}\right) ;\end{matrix}$ $\begin{matrix}\frac{{dC}_{f}}{dt} = - h \left(g, T, σ\right) {C}_{f} - m \left(g, T, σ\right) {C}_{f} - n \left(g, T, σ\right) {C}_{f} - q \left(g, T, σ\right) {C}_{f} \\ + \frac{6 {D}_{g}}{{d}_{g}} {\left.\frac{\partial {C}_{g}}{\partial r}\right|}_{r = {d}_{g} / 2} / {N}_{f} + l \left(g, T, σ\right) {N}_{b} / {N}_{f} ;\end{matrix}$ $\frac{{dC}_{e}}{dt} = - h \left(g, T, σ\right) {C}_{e} - m \left(g, T, σ\right) {C}_{e} + q \left(g, T, σ\right) \frac{{N}_{e}}{{N}_{f}} \left(1-P\right) {C}_{f} - {C}_{e} \frac{dP}{dt} .$ where *a*(*g,T,σ*) represents a bubble nucleation coefficient, *b*(*g,T,σ*) represents a bubble absorption coefficient, *D_{g}* represents a gas diffusion coefficient, *d_{g}* represents a grain diameter, *s* represents a gas generation amount; *e*(*g,T,σ*) represents a re-dissolution coefficient of intragranular bubbles, *h*(*g,T,σ*) represents a re-dissolution coefficient of grain-face (or grain-edge) bubbles, *l*(*g,T,σ*) represents an absorption coefficient of grain-boundary bubbles, *m*(*g,T,σ*) represents a detachment coefficient of grain-face (or grain-edge) bubbles, *n*(*g,T,σ*) represents a direct release coefficient of grain-face bubbles, *q*(*g,T,σ*) represents a migration coefficient of grain-face bubbles to grain edges, *P* represents a release coefficient of grain-edge bubbles, *N_{b}* represents an average number of bubbles per grain, *N_{f}* represents an average number of bubbles per grain face, *Nₑ* represents the number of grain bubbles, *r* represents a distance from a grain center in the grain, that is, *r* in a spherical coordinate system, and *t* represents an evolution time.

In some embodiments, by solving the above bubble evolution governing equation, it is possible to obtain the distribution of fission gas within grains, on grain boundaries and at grain edges, the release amount *wᵣₑₐ* of the fission gas, the induced swelling Δ*V_{α}*, and the porosity distribution *p*(*r̅*).

In some embodiments, the solid fission product behavior unit is specifically used to characterize a behavior of solid fission products generated during the fission process, so as to determine a precipitation phase, a migration behavior and a spatial distribution of the solid fission products of the fuel. The behavior of the solid fission products includes a behavior of strontium, cesium, iodine and lanthanum/actinide fission products, and the behavior of the solid fission products may be controlled by a predetermined evolution equation in embodiments of the present disclosure.

In some embodiments, through the calculation of the solid fission product behavior unit, it is possible to obtain the precipitation phase, the migration behavior and the spatial distribution of the solid fission products of the fuel, which is denoted by *g_{fis}*, including a fuel precipitation phase structure information *str_{fis-pre},* a concentration distribution *C_{fis}* , and a diffusion *D_{fis}* of solid fission products in the fuel. The migration behavior and the spatial distribution *g_{fis}* meet: ${g}_{fis} = {f}_{g - fis} \left({str}_{fis - pre} , {C}_{fis} , {D}_{fis}\right) ;$ where *f_{g-fis}* represents a function for determining a behavior of the solid fission products of the fuel through a numerical method.

In some embodiments, the coupling module 30 is further used to transmit the temperature distribution *T*(*r̅,t*)*,* the stress distribution *σ*(̅*r̅,t*)*,* the fission gas release amount *wᵣₑₐ* and the solid fission product distribution *g_{fis}* of the fuel simulated by the fuel service property sub-module 22 to the material irradiation sub-module 21, so as to perform a multi-scale simulation based on the material and update an irradiation damage behavior of the material under new conditions of temperature, stress, fission gas and solid fission products, thereby improving the accuracy of the changes in the macroscopic mechanical properties or in the geometric integrity properties of the material obtained by simulation.

In some embodiments, the coupling module 30 is further used to: transmit a mechanical property change *g_{fis}* , a geometric integrity parameter Δ*ε_{c}* and a microstructure defect distribution *Nⁱ = f*(*dⁱ,t*) of the material to the temperature distribution unit, the stress distribution unit and the microstructure evolution unit in the fuel service property sub-module 22 to update the service properties of the fuel, thereby improving the accuracy of the change in the service properties of the fuel obtained by simulation.

In some embodiments, FIG. 3 shows a schematic structural diagram of a numerical reactor according to another embodiment of the present disclosure. As shown in FIG. 3, the numerical reactor further includes a data-driven module, which is used to determine a mapping relationship between an actual irradiation result of the reactor, the simulation result of the material irradiation sub-module 21, and the simulation result of the fuel service property sub-module 22.

In some embodiments, as shown in FIG. 3, the numerical reactor further includes a verification module 60, which is used to determine the accuracy of the simulation result of the material irradiation sub-module 21 and the accuracy of the simulation result of the fuel service property sub-module 22 based on the actual irradiation result of the reactor as well as the mapping relationship between the actual irradiation result of the reactor, the simulation result of the material irradiation sub-module 21 and the simulation result of the fuel service property sub-module 22.

In the numerical reactor provided in embodiments of the present disclosure, the verification module 60 may verify the accuracy of the service properties of the fuel and the material according to the actual irradiation result of the reactor as well as the mapping relationship between the actual irradiation result of the reactor, the simulation result of the material irradiation sub-module 21 and the simulation result of the fuel service property sub-module 22, which may help correct parameters of the numerical reactor, improve accuracy and reliability of the calculation result of the numerical reactor, and further avoid insufficient data of the actual irradiation result of the reactor.

In some embodiments, the actual irradiation result of the reactor may include measured data of the reactor and prior physical knowledge.

In some embodiments, the data-driven module 50 is obtained by: acquiring the measured data of the reactor and the prior physical knowledge, where the measured data includes a temperature, a pressure, a flow rate, a neutron flux and a power distribution of the reactor; determining a data-driven module 50 with cross-scale multi-physical feature fusion based on the measured data and the prior physical knowledge; and correcting parameters of the data-driven module 50 according to an output result of the data-driven module 50, the measured data, and the prior physical knowledge.

The data-driven module 50 obtained by the method provided in embodiments of the present disclosure may ensure that the fusion of multi-scale features follows physical laws and has a high reliability.

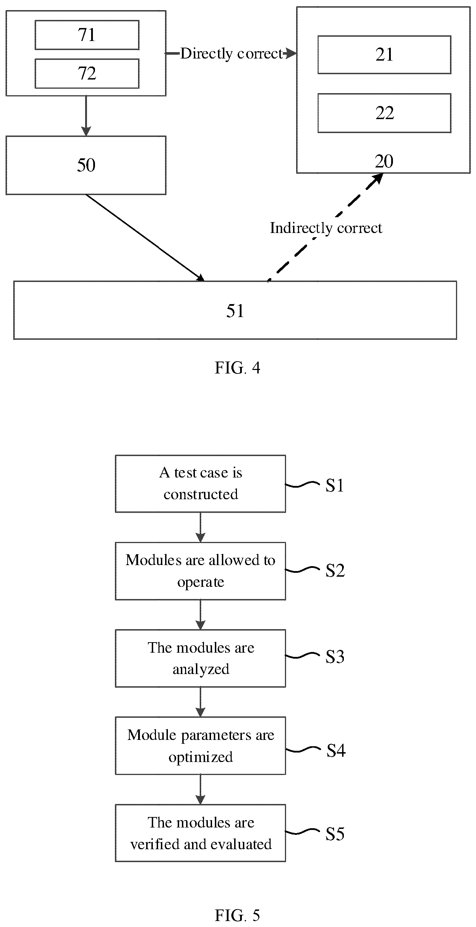

FIG. 4 shows a schematic diagram of correcting parameters of the material irradiation sub-module 21 and the fuel service property sub-module 22 according to an embodiment of the present disclosure. As shown in FIG. 4, on the one hand, it is possible to directly correct the parameters of the material irradiation sub-module 21 and the fuel service property sub-module 22 using measured data 71 and prior physical knowledge 72; on the other hand, it is possible to indirectly correct the parameters of the material irradiation sub-module 21 and the fuel service property sub-module 22 according to a mapping relationship 51 determined by the data-driven module.

In some embodiments, the measured data 71 may include experimental data, actual operation data of the reactor, and the like. When collecting the measured data 71, not only macro-level operation parameters of the reactor are focused on, but also interrelated multi-scale data is collected through multi-scale experimental device and measurement means.

In some embodiments, the measured data 71 of the reactor may include but not be limited to temperature, pressure, flow rate, neutron flux, power distribution, and other key parameters. In order to ensure diversity and representativeness of the data, the measured data 71 may correspond to reactor states under different operating conditions and different working conditions.

In some embodiments, the collected measured data 71 may be cleaned to exclude outliers and erroneous data contained therein. For example, it is possible to perform a noise reduction on the collected measured data 71 to reduce an influence of measurement noise on the data by filtering methods, thereby improving data quality. For another example, it is possible to normalize the collected measured data 71 so that data of different parameters are at the same order of magnitude, which facilitates subsequent analysis and processing.

FIG. 5 shows a schematic diagram of a verification process of the verification module 60 according to an embodiment of the present disclosure. As shown in FIG. 5, the verification module 60 is used to perform step S1 to step S5 as follows.

In S1, a test case is constructed, which includes: directly constructing a test case based on the measured data 71 and the prior physical knowledge 72; indirectly constructing a test case based on the mapping relationship 51 between the actual irradiation result of the reactor, the simulation result of the material irradiation sub-module 21 and the simulation result of the fuel service property sub-module 22 determined by the data-driven module 50.

In S2, modules are allowed to operate, which includes: allowing the material irradiation sub-module 21 and the fuel service property sub-module 22 to operate based on the directly constructed test case and the indirectly constructed test case, so as to obtain the simulation result of the material irradiation sub-module 21 and the simulation result of the fuel service property sub-module 22.

In S3, the modules are analyzed, which includes: performing a comparative analysis to determine differences and deficiencies in the material irradiation sub-module 21 and the fuel service property sub-module 22. For example, the material irradiation sub-module 21 or the fuel service property sub-module 22 may have inaccurate calculations of some specific types, or the module parameters need further optimization. By identifying the differences and deficiencies, it is possible to determine the aspects that need to be corrected.

In S4, module parameters are optimized, which includes: optimizing the parameters of the material irradiation sub-module 21 and the fuel service property sub-module 22 based on the comparative analysis and causes for the differences. It is possible to adjust parameter value ranges, add other key parameters or consider nonlinear relationships to enhance adaptability and prediction ability of the modules. In the optimization process, it is possible to adopt a trial-and-error method to find a good parameter combination through multiple parameter adjustments and operations, or adopt a genetic algorithm for optimizing module parameters to find an optimal combination of module parameters by simulating a biological evolution process, so as to minimize an error between a module prediction value and the measured data 71.

In S5, the modules are verified and evaluated, which includes: after the optimization of module parameters is completed, verifying and evaluating the optimized material irradiation sub-module 21 and fuel service property sub-module 22, so as to ensure that the simulation results obtained based on the corrected material irradiation sub-module 21 and fuel service property sub-module 22 are closer to the measured data 71.

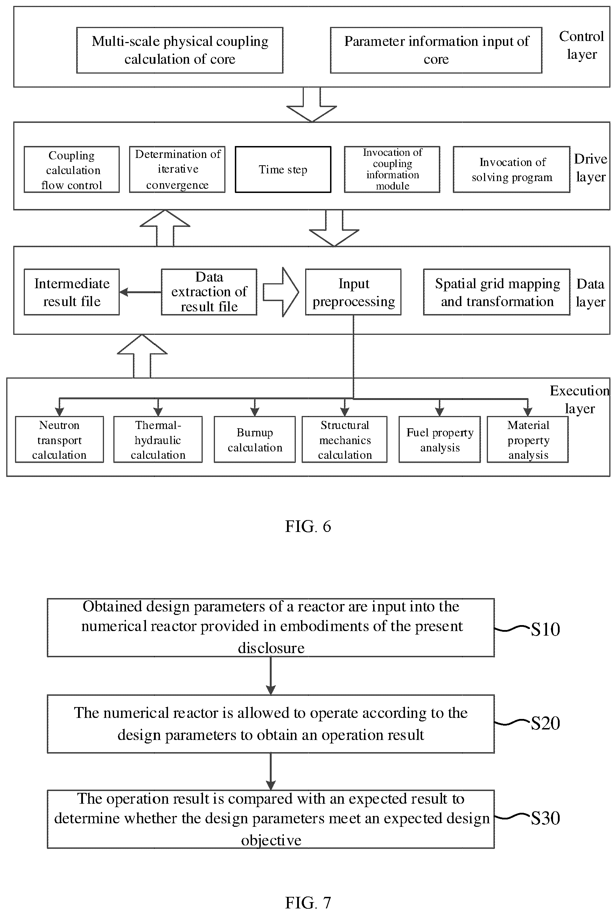

In some embodiments, FIG. 6 shows a schematic diagram of multi-scale physical coupling according to an embodiment of the present disclosure. As shown in FIG. 6, a multi-scale physical coupling module may be constructed using a master-slave architecture based on a neutron transport program, a burnup program, a thermal-hydraulic calculation program, a structural mechanics calculation program, a fuel property analysis program, and a material property analysis program of a single physical process. The multi-scale physical coupling module may be divided into a control layer, a drive layer, a data layer, and an execution layer according to functional requirements.

Establishing representation standards and unified interfaces for grid data and parameters based on the grid type and simulation parameters may help save storage resources and improve access efficiency. An efficient conversion and transfer of parameters based on efficient positioning data index and interpolation algorithm in different spatial discretization forms may help improve the coupling efficiency. Establishing the mapping relationship 51 for a coupling parameter transfer function based on a specific coupling calculation model may help improve the accuracy of coupling parameter transfer. The use of single-level and multi-level time-step control methods may help balance a simulation accuracy and a calculation time.

Embodiments of the present disclosure further provide a method of verifying a reactor design. FIG. 7 shows a schematic flowchart of a method of verifying a reactor design according to an embodiment of the present disclosure. As shown in FIG. 7, the method includes step S10 to step S30.

In S10, obtained design parameters of a reactor are input into the numerical reactor provided in embodiments of the present disclosure.

In S20, the numerical reactor is allowed to operate according to the design parameters to obtain an operation result.

In S30, the operation result is compared with an expected result to determine whether the design parameters meet an expected design objective.

According to the method provided in embodiments of the present disclosure, by inputting the obtained design parameters of the reactor into the numerical reactor provided in embodiments of the present disclosure, it is possible to verify the designed reactor to check whether design values of the reactor meet requirements, and correct the design values according to the operation of the numerical reactor. Furthermore, the above-mentioned numerical reactor is also applicable to verifying materials of in-core components, and further guide research and development of in-core materials according to a verification result.

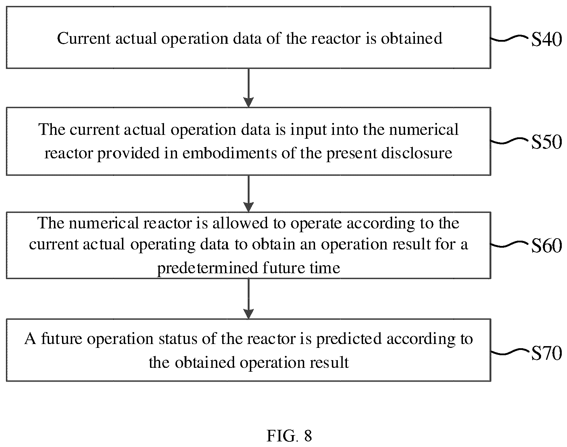

Embodiments of the present disclosure further provide a method of predicting a reactor operation. FIG. 8 shows a schematic flowchart of a method of predicting a reactor operation according to an embodiment of the present disclosure. As shown in FIG. 8, the method includes step S40 to step S70.

In S40, current actual operation data of the reactor is obtained.

In S50, the current actual operation data is input into the numerical reactor provided in embodiments of the present disclosure.

In S60, the numerical reactor is allowed to operate according to the current actual operating data to obtain an operation result for a predetermined future time.

In S70, a future operation status of the reactor is predicted according to the obtained operation result.

The method provided in embodiments of the present disclosure may use data of a reactor that is currently in actual operation. These data may be input into the numerical reactor provided in embodiments of the present disclosure, and the numerical reactor may operate based on these data, so that future data of the reactor may be obtained, that is, a future operation status of the actual reactor may be predicted. According to the prediction, a possible service status of the reactor in the future may be determined, and safety measures may be taken in advance for special situations, thereby improving the reliability of reactor operation.

It may be understood that there is no sequential order of execution for step S30 and step S40 in the embodiment of the present disclosure.

For embodiments of the present disclosure, it should be noted that, without conflicts, embodiments of the present disclosure and features in embodiments may be combined with each other to obtain additional embodiments.

The above are merely specific implementations of the present disclosure, and the scope of protection of the present disclosure is not limited to this. The scope of protection of the present disclosure should be determined by the scope of protection of the claims.

## Claims

1. A numerical reactor, comprising:
a physical-thermal structure module, configured to simulate a neutron field, a temperature field and a stress field between cores of a reactor;
a fuel-material module, configured to simulate a microstructural change of a fuel in the reactor during a fission process and a microstructural change of a material of the reactor; and
a coupling module, configured to couple a neutron field, a temperature field and a stress field of the fuel-material module according to the neutron field, the temperature field and the stress field of the physical-thermal structure module, and configured to couple the neutron field, the temperature field and the stress field of the physical-thermal structure module according to the microstructural change of the material and the microstructural change of the fuel in the fuel-material module.

2. The numerical reactor according to claim 1, wherein the neutron field, the temperature field and the stress field of the fuel-material module are coupled according to the neutron field, the temperature field and the stress field of the physical-thermal structure module by using: ${Φ}_{f} \left(r,t+Δt\right) = {f}_{ϕ} \left({T}_{f}\left(r, t\right),{σ}_{f}\left(r, t\right),{Φ}_{c}\left(r, t\right)\right) ;$ ${T}_{f} \left(r,t+Δt\right) = {f}_{T} \left({T}_{f}\left(r, t\right),{σ}_{f}\left(r, t\right),{Φ}_{f}\left(r, t\right),{T}_{c}\left(r, t\right)\right) ;$ ${σ}_{f} \left(r,t+Δt\right) = {f}_{σ} \left({T}_{f}\left(r, t\right),{σ}_{f}\left(r, t\right),{Φ}_{f}\left(r, t\right),{σ}_{c}\left(r, t\right)\right) ;$ where Φ*_{c}*(*r,t*) represents the neutron field between cores of the physical-thermal structure module, *T_{c}*(*r,t*) represents the temperature field between cores of the physical-thermal structure module, *σ_{c}*(*r,t*) represents the stress field between cores of the physical-thermal structure module, Φ*_{f}*(*r,t*) represents the neutron field of the fuel-material module, *T_{f}*(*r,t*) represents the temperature field of the fuel-material module, *σ_{f}*(*r,t*) represents the stress field of the fuel-material module, Φ*_{f}*(*r,t*+Δ*t*) represents a coupled neutron field of the fuel-material module, *T_{f}*(*r,t+*Δ*t*) represents a coupled temperature field of the fuel-material module, *σ_{f}*(*r,t+*Δ*t*) represents a coupled stress field of the fuel-material module, *r* represents spatial coordinates, *r*≤*r_{c}, r_{c}* represents coordinates of an outer boundary of a cladding of the reactor, *t* represents time, Δ*t* represents a time increment in an iterative calculation, *f_{ϕ}* represents a function for determining the neutron field of the fuel-material module by a numerical method, *f_{T}* represents a function for determining the temperature field of the fuel-material module by a numerical method, and *f_{σ}* represents a function for determining the stress field of the fuel-material module by a numerical method.

3. The numerical reactor according to claim 1, wherein the fuel-material module comprises:
a material irradiation sub-module, configured to simulate a change in properties of the material caused by the microstructural change of the material of the reactor generated after irradiation; and
a fuel service property sub-module, configured to simulate the microstructural change of the fuel in the reactor during the fission process.

4. The numerical reactor according to claim 3, wherein the coupling module is further configured to:
couple service properties of the fuel according to the microstructural change of the material, and couple the properties of the material according to the microstructural change of the fuel.

5. The numerical reactor according to claim 3, wherein the neutron field, the temperature field and the stress field of the fuel-material module and a microstructure of the fuel meet: ${G}_{f} \left(r,t+Δt\right) = {f}_{Gf} \left({Φ}_{f}\left(r, t\right),{T}_{f}\left(r, t\right),{σ}_{f}\left(r, t\right),{G}_{f}\left(r, t\right),{G}_{m}\left(r, t\right)\right) ;$ wherein the neutron field, the temperature field and the stress field of the fuel-material module and a microstructure of the material meet: ${G}_{m} \left(r,t+Δt\right) = {f}_{Gm} \left({Φ}_{f}\left(r, t\right),{T}_{f}\left(r, t\right),{σ}_{f}\left(r, t\right),{G}_{f}\left(r, t\right),{G}_{m}\left(r, t\right)\right) ;$ where *G_{f}*(*r,t+*Δ*t*) represents a coupled microstructure of the fuel, *Gₘ*(*r,t*+Δ*t*) represents a coupled microstructure of the material, Φ*_{f}*(*r,t*) represents the neutron field of the fuel-material module, *T_{f}*(*r,t*) represents the temperature field of the fuel-material module, *σ_{f}*(*r,t*) represents the stress field of the fuel-material module, *G_{f}*(*r,t*) represents the microstructure of the fuel at time *t, Gₘ*(*r,t*) represents the microstructure of the material at time *t,* Δ*t* represents a time increment in an iterative calculation, *f_{Gf}* represents a function for determining the microstructure of the fuel by a numerical method, and *f_{Gm}* represents a function for determining the microstructure of the material by a numerical method.

6. The numerical reactor according to claim 3, wherein the change in the properties of the material comprises a change in macroscopic mechanical properties or in geometric integrity properties of the material;
wherein the material irradiation sub-module is further configured to transmit the change in the macroscopic mechanical properties or in the geometric integrity properties of the material determined by simulation to the fuel service property sub-module; and
wherein the fuel service property sub-module is further configured to correct a change in service properties of the fuel according to the change in the macroscopic mechanical properties or in the geometric integrity properties of the material.

7. The numerical reactor according to claim 6, wherein the service properties of the fuel comprise fission properties and a fission product behavior, the fission properties comprise a temperature and stress distribution, and the fission product behavior comprises a gaseous fission product behavior and a solid fission product behavior;
wherein the fuel service property sub-module is further configured to transmit the temperature and stress distribution, the gaseous fission product and the solid fission product of the fuel determined by simulation to the material irradiation sub-module; and
wherein the material irradiation sub-module is further configured to determine the change in the macroscopic mechanical properties or in the geometric integrity properties of the material according to the temperature and stress distribution, the gaseous fission product and the solid fission product of the fuel.

8. The numerical reactor according to claim 7, wherein the coupling module is further configured to:
couple the service properties of the fuel according to the change in the macroscopic mechanical properties or in the geometric integrity properties of the material.

9. The numerical reactor according to claim 7, wherein the coupling module is further configured to:
couple the macroscopic mechanical properties or the geometric integrity properties of the material according to the temperature and stress distribution, the gaseous fission product and the solid fission product of the fuel.

10. The numerical reactor according to claim 6, wherein the material irradiation sub-module comprises a material neutron field, a material stress field and a material temperature field;
wherein the material neutron field and the material temperature field are configured to simulate a change in the material generated under an irradiation of the material neutron field and a temperature of the material temperature field;
wherein the material stress field and the material temperature field are configured to simulate a change in the material generated under a stress of the material stress field and a temperature of the material temperature field; and
wherein the coupling module is further configured to couple changes in properties of the material according to simulation results of the material neutron field, the material stress field and the material temperature field.

11. The numerical reactor according to claim 10, wherein the coupling module is further configured to couple the material neutron field, the material stress field and the material temperature field of the material irradiation sub-module by changing a generation, diffusion and aggregation process of point defects of the material during a service process; and
wherein the coupling module is further configured to:
obtain a first concentration of the point defects under the material temperature field;
obtain a second concentration of the point defects under the material neutron field and the material temperature field; and
obtain a third concentration of the point defects under the material stress field and the material temperature field.

12. The numerical reactor according to claim 11, wherein a total concentration of the point defects under a coupling of the material neutron field, the material stress field and the material temperature field, the third concentration of the point defects under the material stress field and the material temperature field, the first concentration of the point defects under the material temperature field, and the second concentration of the point defects under the material neutron field and the material temperature field meet: $C = {C}_{1} + {C}_{2} - {C}_{0} ;$ where *C* represents the total concentration of the point defects under the coupling of the material neutron field, the material stress field and the material temperature field, *C*₀ represents the first concentration of the point defects under the material temperature field, *C*₁ represents the second concentration of the point defects under the material neutron field and the material temperature field, and *C*₂ represents the third concentration of the point defects under the material stress field and the material temperature field.

13. The numerical reactor according to claim 12, wherein the coupling module is further configured to:
obtain a first diffusion coefficient of the point defects under the material temperature field;
obtain a second diffusion coefficient of the point defects under the material neutron field and the material temperature field; and
obtain a third diffusion coefficient of the point defects under the material stress field and the material temperature field.

14. The numerical reactor according to claim 13, wherein the second diffusion coefficient of the point defects under the material neutron field and the material temperature field, the first diffusion coefficient of the point defects under the material temperature field, the second concentration of the point defects under the material neutron field and the material temperature field, and the first concentration of the point defects under the material temperature field meet: ${D}_{1} = {D}_{0} \times \frac{{C}_{1}}{{C}_{0}} ;$ where *D*₁ represents the second diffusion coefficient of the point defects under the material neutron field and the material temperature field, *D*₀ represents the first diffusion coefficient of the point defects under the material temperature field, *C*₁ represents the second concentration of the point defects under the material neutron field and the material temperature field, and *C*₀ represents the first concentration of the point defects under the material temperature field.

15. The numerical reactor according to claim 14, wherein a defect diffusion coefficient under the coupling of the material neutron field, the material stress field and the material temperature field, the third diffusion coefficient of the point defects under the material stress field and the material temperature field, the total concentration of the point defects, and the third concentration of the point defects under the material stress field and the material temperature field meet: $D = {D}_{2} \times \frac{C}{{C}_{2}} ;$ where *D* represents the defect diffusion coefficient under the coupling of the material neutron field, the material stress field and the material temperature field, *D*₂ represents the third diffusion coefficient of the point defects under the material stress field and the material temperature field, *C* represents the total concentration of the point defects under the coupling of the material neutron field, the material stress field and the material temperature field, and *C*₂ represents the third concentration of the point defects under the material stress field and the material temperature field.

16. The numerical reactor according to claim 15, wherein the coupling module is further configured to:
control an evolution of defects by using a governing equation for defect reaction, under the material neutron field, the material stress field and the material temperature field, wherein the governing equation is expressed as: $\begin{matrix}\frac{{dC}^{i}}{dt} = {G}^{i} + {\sum }_{j} {J}^{j \to i} - {\sum }_{i} {J}^{i \to k} \\ = {G}^{i} + {\sum }_{j} K \times {C}^{j} \times {C}^{i - j} - {\sum }_{i} K \times {C}^{i} \times {C}^{k - i} \\ = {G}^{i} + {\sum }_{j} n \times \left({D}^{j}+{D}^{i - j}\right) \times {C}^{j} \times {C}^{i - j} - {\sum }_{i} n \times \left({D}^{i}+{D}^{k - 1}\right) \times {C}^{i} \times {C}^{k - i} ;\end{matrix}$ where *i, j* and *k* represent defects of different sizes respectively, *Gⁱ* represents a generation term of the point defect *i* , which is input by the material neutron field, *K* and *n* represent a defect reaction rate constant and a reaction radius respectively, a second term on a right side of the governing equation represents an increase term resulting from an evolution from defect *j* to defect *i* , a third term on the right side of the governing equation represents a decomposition, absorption or disappearance term of defect *i* , *Cⁱ* represents a concentration of defect *i* in the material, *C^{j}* represents a concentration of defect *j* in the material, *C^{i-j}* represents a concentration of defects *i* - *j* in the material, *C^{k-i}* represents a concentration of defects *k-i* in the material, *Dⁱ* represents a diffusion coefficient of defect *i* in the material, *D^{j}* represents a diffusion coefficient of defect *j* in the material, *D^{i-j}* represents a diffusion coefficient of defects *i* - *j* in the material, *D*^{*k*-*i*} represents a diffusion coefficient of defects *k-i* in the material, the concentrations *Cⁱ, Cⁱ⁻ⁱ*, *C^{k-i}* and the diffusion coefficients *Dⁱ, D^{i-j}, D^{k-i}* of the defects are obtained under the coupling of the material neutron field, the material stress field and the material temperature field; and
wherein a defect distribution function for the material under the coupling of the material neutron field, the material stress field and the material temperature field is determined based on an evolution of the point defects, and the change in the macroscopic mechanical properties or in the geometric integrity properties of the material is determined based on the defect distribution function.

17. The numerical reactor according to claim 7, wherein the fuel service property sub-module comprises:
a temperature distribution unit, configured to simulate a change in a fuel temperature distribution of the fuel during a fission reaction;
a stress distribution unit, configured to simulate a change in a fuel stress distribution of the fuel during the fission reaction;
a microstructure evolution unit, configured to simulate a change in the microstructure of the fuel during the fission reaction;
a fission gas behavior unit, configured to simulate a fuel swelling induced by fission gas generated from the fuel during the fission reaction and a change in an amount of gas released into a gas cavity; and
a solid fission product behavior unit, configured to simulate a change in a distribution of solid fission products generated from the fuel during the fission reaction in the fuel.

18. The numerical reactor according to claim 17, wherein the coupling module is further configured to:
couple the fission gas behavior, the solid fission product behavior, the microstructure evolution, a change in a fuel stress field and a change in a fuel temperature field, so as to obtain an internal composition, a structure, a thermal conductivity and a change in geometric integrity of the fuel.

19. The numerical reactor according to claim 18, wherein the fission gas behavior, the solid fission product, the microstructure evolution, the fuel stress field and the fuel temperature field in the fuel service property sub-module are determined by coupling the temperature distribution unit, the stress distribution unit, the microstructure evolution unit, the fission gas behavior unit and the solid fission product behavior unit.

20. The numerical reactor according to claim 19, wherein the coupling module is further configured to:
update the temperature and stress distribution as well as changes in the microstructure, the fission gas and the solid fission product behavior of the fuel in service at predetermined time intervals during a coupling process, wherein the temperature distribution unit, the stress distribution unit, the microstructure evolution unit, the fission gas behavior unit and the solid fission product behavior unit are configured to simulate in sequence during the coupling process.

21. The numerical reactor according to claim 20, wherein the fission gas behavior unit is further configured to characterize a transmission of fission gas, an induced swelling and a release amount of the fission gas in the fuel, so as to obtain a distribution of the fission gas within grains, on grain boundaries and at grain edges, the release amount of the fission gas, the induced swelling, and a porosity distribution; and
wherein the fission gas comprises xenon and krypton.

22. The numerical reactor according to claim 21, wherein the distribution of the fission gas within grains, on grain boundaries and at grain edges and the release amount of the fission gas meet: $\frac{{dw}_{rea}}{dt} = q \left(g, T, σ\right) {C}_{f} P + {C}_{e} \frac{dP}{dt} + n \left(g, T, σ\right) {C}_{f} ;$ where *wᵣₑₐ* represents the release amount of the fission gas, *t* represents a service time of the fuel, *q*(*g,T,σ*) represents a migration coefficient of grain-face bubbles to grain edges, *C_{f}* represents a concentration of grain-boundary bubbles, *P* represents a release coefficient of grain-edge bubbles, *Cₑ* represents a concentration of grain-edge bubbles, and *n*(*g,T,σ*) represents a direct release coefficient of grain-face bubbles.

23. The numerical reactor according to claim 22, wherein the distribution of the fission gas within grains, on grain boundaries and at grain edges and the porosity distribution of the fuel meet: $p \left(\overline{r}\right) = {p}_{0} \left(\overline{r}\right) + p ′ \left({r}_{i}\right) ;$ where *p*(*r̅*) represents the porosity distribution of the fuel, *P*₀(*r̅*) represents an initial porosity distribution of the fuel, and *P'*(*rᵢ*) represents a porosity distribution caused by the fission gas.

24. The numerical reactor according to claim 23, wherein the solid fission product behavior unit is further configured to:
characterize a behavior of solid fission products generated during the fission process, so as to determine a precipitation phase, a migration behavior and a spatial distribution of the solid fission products of the fuel, wherein the behavior of the solid fission products comprises a behavior of strontium, cesium, iodine and lanthanum/actinide fission products, and the behavior of the solid fission products is controlled by a predetermined evolution equation.

25. The numerical reactor according to claim 20, wherein the coupling module is further configured to:
transmit a temperature distribution, a stress distribution, a release amount of fission gas and a distribution of solid fission products of the fuel simulated by the fuel service property sub-module to the material irradiation sub-module, so as to perform a multi-scale simulation based on the material and update an irradiation damage behavior of the material under new conditions of temperature, stress, fission gas and solid fission products.

26. The numerical reactor according to claim 20, wherein the coupling module is further configured to:
transmit a change in mechanical properties, a geometric integrity parameter and a microstructural defect distribution of the material to the temperature distribution unit, the stress distribution unit and the microstructure evolution unit in the fuel service property sub-module, so as to update the service properties of the fuel.

27. The numerical reactor according to any one of claims 3 to 26, further comprising:
a data-driven module, configured to determine a mapping relationship between an actual irradiation result of the reactor, a simulation result of the material irradiation sub-module and a simulation result of the fuel service property sub-module.

28. The numerical reactor according to claim 27, wherein the data-driven module is obtained by:
acquiring measured data of the reactor and prior physical knowledge, wherein the measured data comprises a temperature, a pressure, a flow rate, a neutron flux and a power distribution of the reactor;
determining a data-driven module with cross-scale multi-physical feature fusion based on the measured data and the prior physical knowledge; and
correcting parameters of the data-driven module according to an output result of the data-driven module, the measured data, and the prior physical knowledge.

29. The numerical reactor according to claim 28, further comprising:
a verification module, configured to determine an accuracy of the simulation result of the material irradiation sub-module and an accuracy of the simulation result of the fuel service property sub-module based on the actual irradiation result of the reactor as well as the mapping relationship between the actual irradiation result of the reactor, the simulation result of the material irradiation sub-module and the simulation result of the fuel service property sub-module.

30. A method of verifying a reactor design, comprising:
inputting obtained design parameters of a reactor into the numerical reactor according to any one of claims 1 to 29;
allowing the numerical reactor to operate according to the design parameters to obtain an operation result; and
comparing the operation result with an expected result to determine whether the design parameters meet an expected design objective.

31. A method of predicting an operation of a reactor, comprising:
obtaining current actual operation data of the reactor;
inputting the current actual operation data into the numerical reactor according to any one of claims 1 to 29;
allowing the numerical reactor to operate according to the current actual operation data to obtain an operation result for a predetermined future time; and
predicting a future operation status of the reactor according to the obtained operation result.
